# EUROPÄISCHE PATENTANMELDUNG

(11) **EP 0 600 247 A2**
(43) Veröffentlichungstag der Anmeldung: **08.06.1994**
(21) Anmeldenummer: 93117744.8
(22) Anmeldetag: 02.11.1993
(51) Int. Cl.: C07K 3/00, C12N 13/00, C12N 9/06

(54) **Verfahren zur selektiven Desaktivierung von unerwünschten Proteinen in einem Proteingemisch mittels Mikrowelleneinstrahlung**

(30) Priorität: 05.11.1992 DE 4237373
(71) Anmelder: HOECHST AKTIENGESELLSCHAFT, D-65929 Frankfurt am Main (DE)
(72) Erfinder: Bayer, Thomas Dr., D-65812 Bad Soden/Taunus (DE); Holst, Ulrich, Dr., D-65527 Niedernhausen (DE); Wirth, Uwe, Dr., D-63533 Mainhausen (DE)

(57) **Zusammenfassung**

Proteine in einem Proteingemisch in einer Zellsuspension mit weitgehend intakten Zellen lassen sich vorteilhaft mittels Mikrowelleneinstrahlung selektiv desaktivieren.

## Beschreibung

Aus der DE 40 06 436 ist bereits bekannt, daß eine selektive Aufreinigung von Proteinen mittels Mikrowelleneinstrahlung möglich ist. In der genannten Patentschrift werden dazu allerdings die betreffenden Mikroorganismen zunächst aufgeschlossen und anschließend das erhaltene Proteingemisch mittels Mikrowelleneinstrahlung erhitzt.

Überraschenderweise hat sich nun gezeigt, daß durch eine andere Verfahrensweise deutliche Vorteile erzielt werden können. Diese andere Verfahrensweise ist dadurch gekennzeichnet, daß die Erhitzung mittels Mikrowelleneinstrahlung an einer Suspension mit weitgehend intakten Zellen erfolgt, das bedeutet, daß die erwähnte Erhitzung vor dem Zellaufschluß erfolgt. Wie nicht zu erwarten, erzielt man bei dieser Verfahrensweise eine hohe Selektivität der Denaturierung während gleichzeitig der nachfolgende Zellaufschluß erleichtert wird.

Erfindungsgegenstand ist demzufolge ein Verfahren zur selektiven Desaktivierung von unerwünschten Proteinen in einem Proteingemisch durch Erhitzung mittels Mikrowelleneinstrahlung dadurch gekennzeichnet, daß die Erhitzung mittels Mikrowelleneinstrahlung an einer Suspension mit weitgehend intakten Zellen erfolgt. Weitgehend intakte Zellen bedeutet in diesem Zusammenhang, daß bei dem erfindungsgemäßen Verfahren die Mikroorganismen - von den unvermeidlich bei dem Fermentationsprozeß aufgebrochenen Zellen, die nur einen kleinen Bruchteil ausmachen, abgesehen - in intakter Form vorliegen. Das bedeutet insbesondere, daß bei dem erfindungsgemäßen Verfahren vor der Erhitzung durch Mikrowelleneinstrahlung keine Maßnahmen stattfinden, die zu einem Zellaufschluß führen.

Das erfindungsgemäße Verfahren erfolgt in Abhängigkeit von dem jeweiligen Proteingemisch und der gewünschten (nicht zu desaktivierenden) Proteine bei einer Temperatur in Kombination mit einer jeweiligen Verweilzeit, wobei unter den gewählten Bedingungen die gewünschten Proteine gerade nicht desaktiviert werden die unerwünschten Proteine jedoch weitgehend denaturiert werden. Z.B. im Falle von Trigonopsis variabilis zur Denaturierung von Esterasen und Katalasen unter Erhaltung der D-Aminosäure-Oxidase liegt die optimale Temperatur im Bereich von 60 - 85 °C bei einer Verweilzeit von 10 - 40 Sekunden; besonders bevorzugt ist eine Temperatur von 65 - 75 °C bei einer Verweilzeit von 15 - 25 Sekunden, insbesondere eine Temperatur von ca. 70° C bei einer Verweilzeit von ca. 20 Sekunden. Die Effektivität und Selektivität des erfindungsgemäßen Verfahrens kann weiter gesteigert werden, indem unter genau abgestimmten Bedingungen die betreffende Zellsuspension mehreren Erhitzungszyklen mittels Mikrowelleneinstrahlung unterzogen wird.

Das erfindungsgemäße Verfahren kann mittels unterschiedlicher Mikrowellengeräte betrieben werden. Vorzugsweise wird eine Mikrowellenfrequenz von 0,4 - 24 GHz, insbesondere von 0,4 - 2,5 GHz benutzt. Ganz besonders bevorzugt sind die Mikrowellenfrequenzen von ca. 433 MHz, 915 MHz und 2,45 GHz. Vorzugsweise wird ein Mikrowellen-Durchlauferhitzer benutzt, dem gegebenenfalls eine Heißhaltestrecke nachgeschaltet werden kann. Die Arbeitsparameter (laminare oder turbulente Strömung, Einsatz von Mischern etc.) sind von den jeweils eingesetzten Geräten abhängig und jeweils dahingehend zu optimieren, daß die gesamte zu behandelnde Suspension unter definierten gleichbleibenden Bedingungen unter Vermeidung von Temperatur- und Verweilzeitgradienten erhitzt wird. Details der Geräte- und Verfahrenstechnik finden sich in der obengenannten Patentschritt sowie in der darin zitierten Literatur.

Das erfindungsgemäße Verfahren ist z.B. besonders geeignet zur selektiven Desaktivierung von Esterasen und Katalasen unter Erhaltung der Aktivität von D-Aminosäure-Oxidase in Zellen von Trigonopsis variabilis.

Das erfindungsgemäße Verfahren eignet sich weiterhin besonders für eine ökonomische Vorgehensweise, bei der die Zellsuspension ohne Aufarbeitungsschritte direkt nach der Fermentation wie beschrieben erhitzt wird; in der Kulturbrühe vorhandene Beimengungen und Feststoffe führen überraschenderweise zu keinen Komplikationen.

Nach der erfindungsgemäßen Erhitzung können die Mikroorganismen nach Methoden des Standes der Technik aufgearbeitet werde, wobei der Aufschluß der Zellen durch die Behandlung mit Mikrowellen, wie bereits oben erwähnt, erleichtert ist.

Durch die nachfolgenden Ausführungsbeispiele und durch den Inhalt der Patentansprüche soll die vorliegende Erfindung näher erläutert werden.

### Beispiel

### Versuchsaufbau:

Mittels einer Zahnradpumpe wird die Zellsuspension aus einem gerührten Vorlagegefäß in einen Mikrowellenofen gepumpt. Unmittelbar nach Austritt aus der Mikrowellenzone wird die Produkttemperatur gemessen. Damit die Zellsuspension noch für die gewünschte Zeit bei der eingestellten Temperatur gehalten werden kann, schließt sich eine entsprechend dimensionierte und gut isolierte Heißhaltestrecke an. Anschließend wird die Zellsuspension in einem Kühler auf Raumtemperatur abgekühlt und in einem Auffanggefäß gesammelt.

Als Mikrowellenquelle wird z.B. ein Labor-Mikrowellengerät MLS 1200 der Fa. Büchi verwendet. Dieses Gerät ist zur Produktdurchführung an den Seitenwänden mit je einer Bohrung versehen.

Um höhere Abreicherungsraten zu erzielen kann es sinnvoll sein, die Zellsuspension mehrfach zu erhitzen.

Die nachfolgende Tabelle zeigt die Versuchsergebnisse:

**Tabelle 1**

| Änderung der Enzym-Aktivitäten bei mehrfachem Erhitzen (Lauf 1-3) der Zellsuspension Tₘₐₓ = 70°C, Verweilzeit = 20 sec | | | |
|---|---|---|---|
| | D-Aminosäure-Oxidase [%] | Esterasen [%] | Katalasen [%] |
| Blindwert (vor 1. Lauf) | 100 | 100 | 100 |
| 1. Lauf | 100 | 13,0 | 56 |
| 2. Lauf | 81,8 | 7,3 | 36 |
| 3. Lauf | 82,0 | 5,5 | 28 |

## Patentansprüche

1. Verfahren zur selektiven Desaktivierung von unerwünschten Proteinen in einem Proteingemisch durch Erhitzung mittels Mikrowelleneinstrahlung dadurch gekennzeichnet, daß die Erhitzung mittels Mikrowelleneinstrahlung an einer Suspension mit weitgehend intakten Zellen erfolgt.

2. Verfahren gemäß Anspruch 1, dadurch gekennzeichnet, daß eine Mikrowellenfrequenz von 0,4 bis 24 GHz benutzt wird.

3. Verfahren gemäß den Ansprüchen 1 oder 2, dadurch gekennzeichnet, daß eine Mikrowellenfrequenz von 0,4 bis 2,5 GHz benutzt wird.

4. Verfahren gemäß einem oder mehreren der Ansprüche 1 - 3, dadurch gekennzeichnet, daß das Proteingemisch durch Kultivierung von Trigonopsis variabilis gebildet wurde.

5. Verfahren gemäß Anspruch 4, dadurch gekennzeichnet, daß aus dem Proteingemisch von Trigonopsis variabilis die Esterasen und Katalasen desaktiviert werden bei weitgehender Erhaltung der Aktivität der D-Aminosäure-Oxidase.

6. Verfahren gemäß einem oder mehreren der Ansprüche 1 - 5, dadurch gekennzeichnet, daß die selektive Desaktivierung von unerwünschten Enzymen in einer Zellsuspension erfolgt, die nach der Fermentation keinem Aufbereitungsprozeß unterzogen wurde.
